# EUROPEAN PATENT SPECIFICATION

(11) **EP 1 542 588 B1**
(45) Date of publication and mention of the grant of the patent: **18.06.2008**
(21) Application number: 03769498.1
(22) Date of filing: 26.09.2003
(51) Int. Cl.: A61B 5/117

(54) **METHOD FOR RENDERING FINGERPRINTS VISIBLE**
VERFAHREN ZUM SICHTBARMACHEN VON FINGERABDRÜCKEN
PROCEDE PERMETTANT DE RENDRE VISIBLES DES EMPREINTES DIGITALES

(30) Priority: 27.09.2002 EP 02360278
(43) Date of publication of application: 22.06.2005
(73) Proprietor: EURATOM, B-1049 BRUSSELS (BE)
(72) Inventor: RAY, Ian, 76351 Linkenheim-Hochstetten (DE)
(74) Representative: Ocvirk, Philippe
(86) International application number: PCT/EP2003/050663
(87) International publication number: WO 2004/028367

(56) References cited:
- GB-A- 1 540 147
- US-A- 4 700 657
- US-A- 4 806 380
- US-A- 5 194 289
- US-A- 5 925 332

## Description

### FIELD OF THE INVENTION

The present invention is related to investigation and evidence collecting techniques in connection with fingerprints, and concerns a method for rendering fingerprints visible, especially on uneven and discontinuous substrates.

### BACKGROUND OF THE INVENTION

In spite of modem developments in forensic science involving DNA fingerprinting and the analysis of other human traces, the conventional fingerprint remains the main tool for linking a suspect to the scene of a crime or for identifying with a high certainty the perpetrator of a crime.

Various techniques have been developed for rendering fingerprints visible, enabling them to be recorded photographically. On a flat smooth solid surface, such as a pane of glass or a metal plate, this is relatively easily performed using a fine powder which can be coloring or fluorescent.

A known alternative method is to use cyanoacrylate as a fuming agent (see for example US-4 297 383). Under the correct conditions of temperature and humidity, this agent reacts with the fats left behind in the impression of the fingerprint, to form a polymer. This latter can then be made visible by dusting with a powder or by the use of certain fluorescent dyes. This again works very efficiently with a flat solid surface.

However, difficulties are encountered in the case of uneven or fibrous surfaces such as paper and cloth, because dusting or dying of the latent fingerprint is hindered by the open porous nature of the surface.

This marking operation can be done with some difficulty on paper. But there is no effective solution at the moment for developing fingerprint images on cloth.

If this was possible it would represent a major advance in the investigation and resolution of serious crimes such as murder and assault where contact with clothing is often involved.

GB 1,540,147 describes a method for developing latent fingerprints, wherein the surface presumably bearing the fingerprints is contacted with a reagent comprising small particles associated with a surfactant. In one embodiment, the small particles may comprise particulate silver to allow subsequent intensification of the prints by combination with radioactive elements (e.g. ³⁵S) to produce autoradiographs. Alternatively, the small particles associated with the surfactant may be radioactive powder.

US 5,194,289 relates to a method for labelling an object for its identification. A selected person's fingerprint is applied to an object to be identified, at a predetermined location. Next, the predetermined location is exposed to a vaporous agent comprising vapour of a cyanoacrylate ester. Either the fingerprint or the cyanoacrylate ester bears a detectable amount of an UV sensitive dye. The vaporous agent creates a permanent impression of the fingerprints on the object, which impression is perceptible only in presence of wavelengths of appropriate energy.

### OBJECT OF THE INVENTION

The aim of the present invention is to overcome the afore mentioned limitation and to propose a solution enabling good fingerprint rendering on various materials and surfaces, especially on uneven and discontinuous substrates such as paper, fabric and clothing material.

### SUMMARY OF THE INVENTION

To achieve this goal the present invention concerns a method for rendering fingerprint(s) visible by subjecting or exposing the surface of the substrate presumably bearing the fingerprint(s), or at least a corresponding region of said surface, to a fuming agent, under adapted conditions.

The inventive method is mainly characterized in that said fuming agent consists of an agent able to react with substances of the fingerprints and labelled with a radioactive isotope able to mark a film-like material coated or impregnated with an adapted reactive substance, and in that said method also consists in placing a portion of such a film-like material in a close position to said surface or surface region and, possibly, submitting said exposed portion of said film-like material to a developing or visualisation process. Said latter step is not required if the film material is self developing.

The size of the used film-like material will be of course equivalent to the area of the substrate with suspected fingerprints.

According to a preferred embodiment of the invention, the fuming agent is a cyanoacrylate based agent labelled with a radioactive isotope and said film-like material comprises advantageously a photographic film, said latter being for example usually available film Material.

Although many radioactive isotopes could be used a natural harmless isotope such as radioactive ¹⁴C is preferred.

The present method is implemented with a fuming agent that is labelled with a radioactive isotope. The radioactive labelling of the fuming agent is preferably done by substitution of at least one element of the fuming agent by a radioactive isotope. The fuming agent is thus essentially unaltered and is capable of marking a film-like material (e.g. photographic film). Alternatively, the radiolabelling could be done by addition, i.e. by adding to the molecule of the fuming agent a radioactive group or element.

In order to obtain a high resolution in terms of reproduction of the fingerprint(s) details beared by the analysed surface of the substrate, said material is advantageously brought into close or intimate contact with the surface or surface region bearing the fingerprint(s), during a given exposure time depending on the used film-like material.

Thus, according to a preferred materialisation of the inventive method, said latter proposes to label cyanoacrylate with radioactive ¹⁴C, and to use this labelled cyanoacrylate as a fuming agent for fingerprints on difficult substrates. This eliminates the step of developing the latent fingerprint(s) with soot or dyes, since said latent fingerprint(s) can then be imaged directly by placing a photographic film in close contact with the substrate.

Although cyanoacrylate based agents are preferred, other types of molecules able to react with the substances of the fingerprints may also be used, as long as they are adapted to the fuming procedure.

Furthermore, it will be appreciated that the present method may be generally implemented with any fuming agent that is able to react with at least one of the substances contained in the fingerprints, in particular with fatty substances and/or amino acids and/or riboflavin, and that is adapted to the fuming procedure.

The method can also be applied to the imaging of fingerprints on curved or irregular surfaces.

The present invention also concerns the use of radioactively-labelled cyanoacrylate as a fuming agent in a method for rendering fingerprints visible, in connection with a film-like imaging material, especially within the context of the before described method.

Preferably, the radioactive labelling additive is ¹⁴C.

The present invention is, of course, not limited to the preferred embodiments described and represented herein, changes can be made or equivalents used without departing from the scope of the invention.

### DETAILED DESCRIPTION OF A PREFERRED EMBODIMENT

A preferred embodiment of the method in according with the invention will now be described in detail, by way of example, wherein the fuming agent is a cyanoacrylate based agent radiolabelled with ¹⁴C.

In order to reveal fingerprints, the surface of a substrate presumably bearing the fingerprint(s) is subjected to the fuming agent. This is typically done by fuming the substrate with vapours of the fuming agent. The fuming agent deposits onto the substrate and reacts with the with the fatty substances of the fingerprints.

According to the present embodiment, the fuming agent is a cyanoacrylate based agent that is radiolabelled with radioactive ¹⁴C.

After subjecting the substrate to the fuming agent, a film-like material is placed closed to or in contact with the surface onto which the fuming agent has been applied, during a given exposure time. The activity of the ¹⁴C included in the fuming agent molecules will cause the marking the film-like material and thus form an image of the fingerprints on the film-like material. If required, the exposed film-like material is then subjected to a developing or visualisation operation.

As mentioned, the surface of a substrate presumably bearing the fingerprint(s) is subjected to vapours of the fuming agent, i.e. the ¹⁴C labelled cyanoacrylate based agent. This is typically done by fuming the substrate in a fuming vessel (or fuming cupboard) under controlled conditions of temperature and humidity. Typically, for cyanoacrylate the evaporation temperature is 130°C and the relative humidity is about 60 %. Samples are hung in the fuming vessel for various times depending on their form and surface area. Commercially available fuming vessels can be used to implement the present method. An advantage of employing a ¹⁴C radiolabelled cyanoacrylate based fuming agent is that it allows to implement the present method with standard equipment and that fuming can be carried out under conditions similar to those used with a conventional cyanoacrylate fuming agent.

Regarding now the labelling of the cyanoacrylate based agent, this is typically carried out by substitution during the synthesis of the fuming agent. This is preferably done by replacing some of the elements of the cyanoacrylate based agent by radioactive isotopes of the same elements. In the present embodiment, the synthesis of the cyanoacrylate is carried out in such a way that a C₂H₅ group of the cyanoacrylate based molecule is replaced by the same group but that includes radioactive ¹⁴C.

When using ¹⁴C as radiolabeling isotope, the film-like material is preferably a photographic film. The choice of film sensitivity, exposure time and distance at which the photographic film is positioned with regard to the fingerprints on the substrate depend on the desired resolution in terms of reproduction of the fingerprints. If necessary, the photographic film may even be brought into close or intimate contact with the substrate.

## Claims

1. Method for rendering fingerprint(s) visible by subjecting or exposing the surface of a substrate presumably bearing the fingerprint(s), or at least a corresponding region of said surface, to a fuming agent, under adapted conditions, said fuming agent consisting of an agent able to react with substances of the fingerprints and **characterized in that** it is labelled with a radioactive isotope able to mark a film-like material coated or impregnated with an adapted reactive substance, and **in that** said method also consists in then placing a portion of such a film-like material in a close position to said surface or surface region and submitting said exposed portion of said film-like material to a developing or visualisation process.

2. Method according to claim 1, **characterized in that** the fuming agent is a cyanoacrylate based agent.

3. Method according to claim 1 or 2, **characterized in that** said film-like material comprises a photographic film.

4. Method according to any of claims 1 to 3, **characterized in that** said radioactive isotope is ¹⁴C.

5. Method according to claim 1, **characterized in that** said fuming agent is prepared in such a way that some of its elements are substituted by their radioactive isotope.

6. Method according to claim 5, **characterized in that** said fuming agent is radiolabelled with radioactive ¹⁴C.

7. Method according to any one of the preceding claims, **charaterized in that** said fuming agent is able to react with fatty substances and/or amino acids and/or riboflavin of said fingerprints.

8. Method according to any one of claims 1 to 7, **characterized in that** said film-like material is brought into close contact with the surface or surface region bearing the fingerprint(s), during a given exposure time.

9. Use of radioactivety-labelled cyanoacrylate as a fuming agent in a method for rendering fingerprints visible, in connection with a film-like imaging material.

10. Use according to claim 9, **characterized in that** the radioactive label is ¹⁴C.

## Patentansprüche

1. Verfahren zum Sichtbarmachen eines Fingerabdrucks (von Fingerabdrücken) indem die Oberfläche eines Substrats, die mutmaßlich den Fingerabdruck (die Fingerabdrücke) aufweist, oder wenigstens eine entsprechende Region der Oberfläche unter angepassten Bedingungen einem Bedampfungsmittel ausgesetzt oder damit in Kontakt gebracht wird, wobei das Bedampfungsmittel aus einem Mittel besteht, das in der Lage ist, mit Substanzen der Fingerabdrücke zu reagieren, und **dadurch gekennzeichnet, dass** es mit einem radioaktiven Isotop markiert ist, das in der Lage ist, ein filmartiges Material, welches mit einer angepassten reaktionsfähigen Substanz beschichtet oder imprägniert ist, zu markieren, und dass das Verfahren auch das darauffolgende Anordnen eines Abschnitts eines derartigen filmartigen Materials an einer Position nahe bei der Oberfläche oder der Oberflächenregion und das Unterziehen des belichteten Abschnitts des filmartigen Materials einem Entwicklungs- oder Visualisierungsprozess umfasst.

2. Verfahren nach Anspruch 1, **dadurch gekennzeichnet, dass** das Bedampfungsmittel ein Mittel auf Cyanacrylatbasis ist.

3. Verfahren nach Anspruch 1 oder 2, **dadurch gekennzeichnet, dass** das filmartige Material einen fotografischen Film umfasst.

4. Verfahren nach irgendeinem der Ansprüche 1 bis 3, **dadurch gekennzeichnet, dass** das radioaktive Isotop ¹⁴C ist.

5. Verfahren nach Anspruch 1, **dadurch gekennzeichnet, dass** das Bedampfungsmittel derart hergestellt wird, dass einige seiner Elemente durch deren radioaktives Isotop substituiert werden.

6. Verfahren nach Anspruch 5, **dadurch gekennzeichnet, dass** das Bedampfungsmittel mit radioaktivem ¹⁴C radioaktiv markiert ist.

7. Verfahren nach irgendeinem der vorhergehenden Ansprüche, **dadurch gekennzeichnet, dass** das Bedampfungsmittel in der Lage ist, mit Fettsubstanzen und/oder Aminosäuren und/oder Riboflavin aus den Fingerabdrücken zu reagieren.

8. Verfahren nach irgendeinem der Ansprüche 1 bis 7, **dadurch gekennzeichnet, dass** das filmartige Material eine bestimmte Belichtungszeit lang in nahen Kontakt mit der Oberfläche oder Oberflächenregion, welche den Fingerabdruck (die Fingerabdrücke) aufweist, gebracht wird.

9. Verwendung von radioaktiv markiertem Cyanacrylat als Bedampfungsmittel bei einem Verfahren zum Sichtbarmachen von Fingerabdrücken, in Verbindung mit einem filmartigen Bilddarstellungsmaterial.

10. Verwendung nach Anspruch 9, **dadurch gekennzeichnet, dass** die radioaktive Markierung ¹⁴C ist.

## Revendications

1. Procédé pour rendre une empreinte digitale ou des empreintes digitales visibles en soumettant ou en exposant la surface d'un substrat supposé porter l'empreinte digitale ou les empreintes digitales, ou au moins une région correspondante de ladite surface, à un agent de fumigation, sous des conditions adaptées, ledit agent de fumigation consistant en un agent apte à réagir avec les substances des empreintes digitales et
**caractérisé en ce qu'**il est marqué avec un isotope radioactif apte à marquer un matériau sous forme de film revêtu ou imprégné avec une substance réactive adaptée, et **en ce que** ledit procédé consiste également à placer ensuite une portion d'un tel matériau sous forme de film dans une position à proximité de ladite surface ou région de surface et à soumettre ladite portion exposée dudit matériau sous forme de film à un processus de développement ou de visualisation.

2. Procédé selon la revendication 1, **caractérisé en ce que** l'agent de fumigation est un agent à base de cyanoacrylate.

3. Procédé selon la revendication 1 ou 2, **caractérisé en ce que** ledit matériau sous forme de film comprend un film photographique.

4. Procédé selon l'une quelconque des revendications 1 à 3, **caractérisé en ce que** ledit isotope radioactif est le ¹⁴C.

5. Procédé selon la revendication 1, **caractérisé en ce que** ledit agent de fumigation est préparé de telle manière que certains de ses éléments sont remplacés par leur isotope radioactif.

6. Procédé selon la revendication 5, **caractérisé en ce que** ledit agent de fumigation est marqué avec du ¹⁴C radioactif.

7. Procédé selon l'une quelconque des revendications précédentes, **caractérisé en ce que** ledit agent de fumigation est apte à réagir avec les substances grasses et/ou les acides aminés et/ou la riboflavine desdites empreintes digitales.

8. Procédé selon l'une quelconque des revendications 1 à 7, **caractérisé en ce que** ledit matériau sous forme de film est mis en contact étroit avec la surface ou région de surface portant l'empreinte digitale ou les empreintes digitales, pendant un temps d'exposition donné.

9. Utilisation de cyanoacrylate marqué radioactivement comme agent de fumigation dans un procédé pour rendre des empreintes digitales visibles, en connexion avec un matériau d'imagerie sous forme de film.

10. Utilisation selon la revendication 9, **caractérisé en ce que** le marqueur radioactif est le ¹⁴C.
